# EUROPEAN PATENT APPLICATION

(11) **EP 4 796 093 A1**
(43) Date of publication of application: **26.08.2026**
(21) Application number: 25220234.6
(22) Date of filing: 02.12.2025
(51) Int. Cl.: A61F 13/551, B65D 85/07, B32B 5/02, B32B 27/12, B32B 27/32

(54) **PACKAGING FOR ABSORBENT ARTICLES**

(30) Priority: 21.02.2025 EP 25159441
(71) Applicant: Ontex BV, 9255 Buggenhout (BE); Ontex Group NV, 9320 Erembodegem (BE)
(72) Inventor: LAMBERTZ, Christina, 56566 Neuwied (DE); JANSEN, Bart, 2640 Mortsel (BE)
(74) Representative: Macchetta, Andrea

(57) **Abstract**

A package comprising a bag and a stack of absorbent articles wherein the stack of absorbent articles is arranged in the bag, wherein the bag is made of a composite material, said composite material comprising, preferably consisting essentially of, even more preferably consisting of, a film and at least a layer of nonwoven fabric, wherein the film forms an inner layer of the bag for contact with the absorbent articles, and the nonwoven fabric forms an outer layer of the bag, and wherein the composite material is substantially free of an adhesive between said film and said nonwoven.

## Description

### TECHNICAL FIELD

The present disclosure pertains to the technical field of absorbent articles, and more in particular to packages that for example include a bag and at least one absorbent article, preferably a stack of absorbent articles. The present disclosure also pertains to methods for manufacturing such packages for a stack of absorbent articles.

Absorbent articles for use herein preferably are for personal hygiene (such as baby or adult diapers, pants or incontinence briefs or pads, as well as feminine hygiene article such as liners). Most prefered abosrbent articles herein are diapers and/or pants, preferably baby diapers and/or pants.

### BACKGROUND

In recent years, the demand for eco-friendly absorbent articles has grown significantly, driving innovation towards the use of more sustainable materials and reducing reliance on chemicals. Compared to traditional disposable absorbent articles, modern alternatives increasingly prioritize environmental responsibility without compromising performance.

A key aspect of absorbent article manufacturing is the packaging, which is predominantly made from plastic materials. Plastic packaging offers numerous advantages, including flexibility for various shapes and sizes, ease of sealing, and extensive possibilities for coloring and branding. These attributes make plastic a preferred choice for packaging absorbent articles.

While non-plastic alternatives, such as paper-based packaging, exist and are considered more environmentally friendly, they often lack the same level of flexibility, durability, and usability as plastic. Consequently, despite the industry's sustainability efforts, plastic remains the dominant material. However, there is a growing need to reduce the carbon footprint of packaging materials without sacrificing their functional and aesthetic qualities. Additionally, conventional plastic packaging, being synthetic in nature, often has a less visually appealing and less tactilely pleasant feel, which may be perceived as undesirable by consumers.

Thus, there remains a need for improved packaging solutions that balance sustainability with the practical benefits of traditional plastic packaging while providing a tactile experience of enhanced softness-one that may align with the softness attributes of the absorbent articles contained within the package.

### SUMMARY

In a first aspect the disclosure relates to a package comprising a bag and a stack of absorbent articles wherein the stack of absorbent articles is arranged in the bag, wherein the bag is made of a composite material, said composite material comprising, preferably consisting essentially of, even more preferably consisting of, a film and at least a layer of nonwoven fabric, wherein the film forms an inner layer of the bag for contact with the absorbent articles (), and the nonwoven fabric forms an outer layer of the bag, and wherein the composite material is substantially free of adhesive layer between said film and said nonwoven, preferably wherein the film and nonwoven are substantially co-formed.

In a highly preferred aspect, the film has a basis weight of less than 10 gsm, preferably less than 8 gsm, more preferably from 1 gsm to 5 gsm, even more preferably from 2gsm to 4 gsm, even more preferably about 3 gsm.

In a further highly preferred aspect, the film and the nonwoven a co-formed by extrusion.

In a further aspect the disclosure relates to a method for manufacturing a package for a stack of absorbent articles herein and comprising an open end and a first closed end, the method comprising the steps of: i. arranging the stack of absorbent articles through the open end, and ii. sealing a sealing portion of the open end to obtain a second closed end.

In a further aspect the disclosure relates to a method for manufacturing a package for a stack of absorbent articles, the method comprising the, preferably sequential, steps of:
i. co-forming, preferably co-extruding, a film and nonwoven to form a composite material;
ii. shaping the composite material into a bag having an open end and a first closed end;
iii. filling the bag with absorbent articles through the open end; and
iv. sealing the open end to form a second closed end, such to contain the absorbent articles between first and second closed ends.

### BRIEF DESCRIPTION OF THE FIGURES

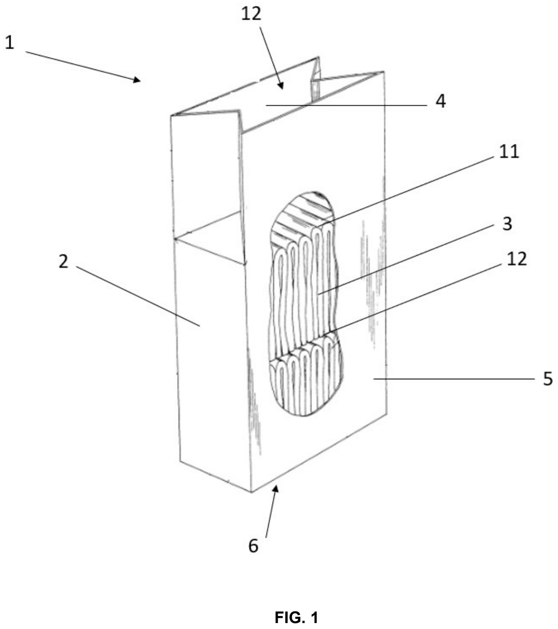

**FIG. 1** is a perspective view of a package according to an embodiment of the present disclosure.

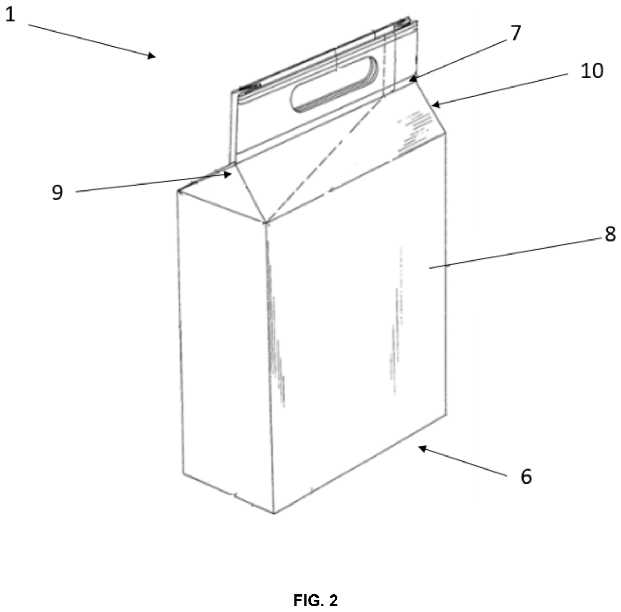

**FIG. 2** is a perspective view of an package according to an embodiment of the present disclosure in an open state and with a partial cut-way section.

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed disclosure. However, it is to be understood that the value to which the modifier "about" refers is itself also specifically disclosed.

The expression "% by weight" (weight percent), here and throughout the description unless otherwise defined, refers to the relative weight of the respective component based on the overall weight of the formulation.

The recitation of numerical ranges by endpoints includes all numbers and fractions subsumed within that range, as well as the recited endpoints unless otherwise stated.

As used herein, the term "absorbent article" refers to disposable devices such as infant or adult diapers or pads, pants, training pants, and the like which are placed against or in proximity to the body of the wearer to absorb and contain the various exudates discharged from the body. Typically these articles comprise a topsheet, backsheet, an absorbent core and optionally an acquisition system (which may be comprised of one or several layers) and typically other components, with the absorbent core normally placed between the backsheet and the acquisition system or topsheet.

A "nonwoven web" as used herein means a manufactured sheet, web or batt of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or man-made origin and may be staple or continuous filaments or be formed in situ. Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yarn). Nonwoven webs can be formed by many processes such as meltblowing, spunbonding, solvent spinning, electrospinning, carding and airlaying. The basis weight of nonwoven webs is usually expressed in grams per square meter (g/m2 or gsm).

The terms "comprise," "comprising," and "comprises" are open ended terms, each specifies the presence of what follows, e.g., a component, but does not preclude the presence of other features, e.g., elements, steps, components known in the art, or disclosed herein. These terms based on the verb "comprise" should be read as encompassing the narrower terms "consisting of" which excludes any element, step, or ingredient not specified and "consisting essentially of" which limits the scope of an element to the specified materials or steps and those that do not materially affect the way the element performs its function. Any preferred or exemplary embodiments described below are not limiting the scope of the claims, unless specifically indicated to do so. The words "typically", "normally", "advantageously" and the likes also qualify elements which are not intended to limit the scope of the claims unless specifically indicated to do so.

By "absorbent material" it is meant a material which has some absorbency property or liquid retaining properties, such as SAP, cellulosic fibers as well as synthetic fibers, most preferably is selected from the group consisting of SAP, cellulose (or cellulosic) fibers, and mixtures thereof. Herein, absorbent materials in the form of fibrous absorbent materials have been found to be useful. These fibrous absorbent materials can comprise or consist of natural fibers, e.g. cellulosic fibers as well as synthetic fibers. Typically, glues used in making absorbent cores have no absorbency properties and are not considered as absorbent material.

As used herein, the term "absorbent core" refers to the component or components of the article having the most absorbent capacity and comprising an absorbent material and optionally a core wrap enclosing the absorbent material. The term "absorbent core" does not include the acquisition-distribution system or layer or any other component of the article which is not either integral part of the core wrap or placed within the core wrap. The core may consist essentially of, or consist of, a core wrap, absorbent material as defined below and glue enclosed within the core wrap.

As used herein "substantially free of adhesive" means less than 20gsm, preferably less than 15gsm, even more preferably less than 10gsm, even more preferable less than 5gsm, most preferably about 0gsm, of adhesive.

As used herein "essentially free" (of polyethylene) means a material, component, element, or product that contains polyethylene in quantities so minimal that its presence is considered insignificant in terms of both mass and functional impact. The polyethylene content is hence typically below detection limits using standard analytical techniques, and/or it constitutes a negligible fraction of the overall composition, having no meaningful influence on the material's physical, chemical, or performance characteristics. In practical terms, such a material, component, element, or product may be effectively considered polyethylene-free.

The term "opacity", as used herein, is intended to refer to the degree to which materials resist transmission therethrough of rays of light. "Opacifiers" are therefore agents known in the art which are capable of imparting additional opacity to materials to which they are added, incorporated, or applied.

### THE PACKAGE

The package (1) comprises a bag (2) and a stack of absorbent articles (3) wherein the stack of absorbent articles is arranged in the bag, wherein the bag (2) is made of a composite material, said composite material comprising, preferably consisting essentially of, even more preferably consisting of, a film and at least a layer of nonwoven fabric, wherein the film forms an inner layer (4) of the bag (2) for contact with the absorbent articles (3), and the nonwoven fabric forms an outer layer (5) of the bag (3), and wherein the composite material is substantially free of an adhesive layer between said film and said nonwoven, preferably wherein the film and nonwoven are substantially co-formed. Advantageously this allows to not only reduce the use of materials (such as adhesives) for improved sustainability but further allows to reduce the overall basis weight of the film and composite material so as to provide overall benefits towards scope 3 typer emissions. Moreover, a sensory experience for the user is provided for by the nonwoven surface which in absence of adhesive in state of the art lamination processes is able to convey a softer perception to the touch.

The stack of absorbent articles (3) may comprise one or more, preferably disposable, absorbent articles. The absorbent articles preferably being selected from diapers, pants, briefs, liners, and towels.

Preferably, the film and nonwoven are co-formed, preferably extrusion co-formed, more preferably such that the nonwoven directly adjoins the film. By "directly" it is intended in absence of additional bonding layers like adhesives. Advantageously this permits the avoidance of adhesive coatings applied therebetween which allows to reduce overall basis weight of the material, CO2 emissions per piece and also potential risks of undesirable adhesive migration particularly when using lower basis weights of nonwovens.

An intermediate layer may be interposed between the film layer and the nonwoven layer. Good joining of the intermediate layer with the film and nonwoven layers results when the intermediate layer is bonded to the said layers both in molten state and thereafter when the structure has been solidified. The interlayer adhesion strength should be, at least, at the level of melt strength of the strongest layer; insufficient adhesion level could only give moderate improvements in draw-down and quickly produces interfacial instabilities by partial or total layer delamination caused by a significant difference in rheological behaviour between layers.

Preferably, the intermediate layer is obtained in one or more of the following ways: (i) The intermediate layer can be formed by chemical interaction between the film and nonwoven layers. In this embodiment, the polymers of the film and nonwoven may be selected to be mutually compatible, meaning that in a molten state and after solidification of the multilayer structure, they form a continuous phase together. Under extrusion temperatures, both the film polymers and the nonwoven polymers may form a blend matrix, thereby forming the intermediate layer during the extrusion process, particularly when they come into contact under heat at a common die; and/or (ii) The intermediate layer can be formed by chemical interaction between the film and nonwoven polymers, provided that the film layer and/or the nonwoven layer also contain a dispersed adhesive material (this being different from the use of adhesive as additional bonding layer as for example in traditional lamination and coating techniques). Under extrusion temperatures, the adhesive material may blend with the film polymer and/or the nonwoven polymer. The adhesive material dispersed in this manner may enhance compatibility between the film and nonwoven polymers. Depending on the degree of compatibility, the adhesive material can be added in an amount ranging from 0.5% to 10% by weight of the total polymer weight of the film and nonwoven layers, when the polymers tend to blend homogeneously in the molten state, or from 10% to 60% by weight when the polymers of the film and nonwoven layers do not blend homogeneously in the molten state.

In an embodiment, the co-formed film is a multi-layered structure and may comprise two layers: one composed of an extensional thinning behavior polymer, optionally containing a dispersed adhesive material, and another composed of an extensional thickening behavior polymer, optionally containing a dispersed adhesive material. Preferably, the same adhesive material can also be used for bonding the layered structure onto the nonwoven layer, thereby eliminating the need for a "distinct" adhesive material for bonding the film to the nonwoven.

The nonwoven fabric may comprise, preferably consists of, one or more layers; and wherein the nonwoven fabric has a basis weight from 5 to 50 gsm, preferably from 5 to 40 gsm, more preferably from 6 to 30 gsm, even more preferably from 7 to 20 gsm, most preferably from 8 to 10 gsm.

The film may have a basis weight of less than 10 gsm, preferably less than 8 gsm, more preferably from 1 gsm to 5 gsm, even more preferably 2 gsm to 4 gsm, even more preferably about 3 gsm. Surprisingly, such co-forming process has been found to be particularly useful when further reducing the basis weight of the film materials. Advantageously, such low basis weight not only brings advantages with regards to less material usage and waste but further also brings a significant positive impact on scope 3 emissions reduction.

Preferably, the film (including all its layers in cases of a multilayered film) has a total thickness of less than 2.5 µm, preferably less than 2 µm, even more preferably from 0.5 µm to 1.5 µm. Especially in these embodiments with low film thickness (which allow for significantly reduced material usage and surprisingly found to, in combination with the nonwovens herein, provide for the desired mechanical performance) it is preferable that the film comprises an opacifier such that the opacity index of the film is at least 35%. In particular the opacity index may range from 40% to 100%, in particular from 45% to 95%, preferably from 50% to 85%, or from 65% to 80%. If the opacity is too low, the film material may appear too thin and provide undesirable see-though/translucency through which products contained inside could be viewable. If the opacity it too high, the amount of opacifier necessary may need to be increased to a level that would start to impact things such as mechanical performance of the film, especially when operating at extremely low basis weights. A balance allows to then use the opacity of the nonwoven to provide the ultimate increase in overall opacity that is desirable to ensure consumer acceptance on shelf.

For example, whiteness, brightness, and/or opacity may be provided to the film by the inclusion of opacifiers/colorants such as TiO2 (titanium dioxide), or sodium aluminum silicate, and optical brighteners on or in the material of the films themselves. Opacifiers used in the films according to the above embodiment preferably are not contained in an amount of more than 25%wt, preferably from 0.01%wt to 10%wt, by weight of the film composition.

The nonwoven may comprise fibers selected from the group consisting of polyolefin fibers, polyester fibers, and mixtures thereof; preferably wherein the nonwoven comprises fibers selected from polyethylene fibers, polypropylene fibers, polylactic acid fibers, and/or Polyethylene terephthalate fibers.

The nonwoven is preferably selected from the group consisting of spunbond, meltblown, and carded nonwovens, and mixtures thereof.

Preferably, the nonwoven comprises one or more spunbond nonwovens and/or meltblown nonwovens. Spunbond nonwovens provide excellent softness, flexibility, and drapability, contributing to a more sensory experience by-touch for the user while also offering good tensile strength and abrasion resistance. Meltblown nonwovens, with their fine fiber structure and dense network, enhance the mechanical integrity of the composite material by improving tear resistance, puncture strength, and barrier properties. These materials offer a balance of strength, flexibility, and processability, making them suitable for use in composite structures where both durability and lightweight properties are required.

More preferably, the nonwoven is a multilayer nonwoven comprising spunbond and meltblown nonwovens, such as spunbond-meltblown (SM), spunbond-meltblown-spunbond (SMS), spunbond-spunbond-meltblown-spunbond (SSMS), and the like. These multilayer structures leverage the complementary properties of spunbond and meltblown layers, resulting in improved mechanical integrity while maintaining a low basis weight and softness.

The film preferably comprises a copolymer of ethylene and methyl acrylate and preferably a thermoplastic polyester elastomer. A suitable copolymer for extrusion is for example commercially available Elvaloy AC1125 (a DOW-polymer ELVALOY^{™} AC 1125 Acrylate Copolymer manufactured and sold by Dow Inc.). A suitable thermoplastic polyester elastomer for extrusion is for example commercially available Hyrtel G5544 (manufactured and sold by the Celanese Corporation under its commercial brand Hytrel^{®}). Advantageously this allows for a reduced basis weight whilst at the same time retaining mechanical performance and resistance to tear.

Preferably, the film is essentially free of polyethylene. Without wishing to be bound by theory, the use of polyethylene, as is customary in state-of-the-art film formation for packaging films of absorbent articles and/or impermeable backsheets of absorbent articles, presents significant limitations, particularly in achieving substantial reductions in the basis weight of the resulting films below 10 gsm or even below 14 gsm while maintaining acceptable mechanical performance. Specifically, polyethylene-based films tend to exhibit inherent challenges in balancing reduced material usage with adequate tensile strength, puncture resistance, and flexibility. The absence of polyethylene in the film formulation allows for the incorporation of alternative polymeric compositions and processing techniques, such as coextrusion with elastomeric or semi-crystalline polymers, which can enhance film performance at lower basis weights.

The composite material may have a total basis weight of from 7 gsm to 30 gsm, preferably from 8 gsm to 25 gsm, even more preferably from 9 gsm to 20 gsm, even more preferably from 10 gsm to 15 gsm, even more preferably from 11 gsm to 14gsm. A lower basis weight contributes to material savings, reduced environmental footprint, and improved package flexibility, while still ensuring adequate barrier properties and mechanical integrity especially when in combination with significantly reduced film basis weight as described herein, a coextruded nonwoven of the above indicated basis weight ranges cooperates synergistically in providing improved mechanical performance of the composite material.

The bag (2) may have a first closed end (6), a second closed end (7), and a peripheral wall (8) between said bottom and said top. The design of the bag (2) provides a structurally stable enclosure for the absorbent articles, reducing potential deformation or damage during storage and transport.

In an embodiment, the peripheral wall (8) comprises a front wall, a rear wall opposite said front wall, a first side wall, and a second side wall opposite said first side wall, and wherein, at a top position, the first and the second side wall are folded inwardly such that two first wing portions (9) are formed where the top joins the first side wall and such that two second wing portions (10) are formed where the top joins the second side wall. This folding configuration enhances compactness and improves structural integrity, facilitating efficient stacking and handling of packaged absorbent articles.

Preferably, the stack of absorbent articles is arranged such that each absorbent article (3) extends in an upright direction between the first closed end (6) and the second closed end (7). This orientation maximizes spatial efficiency within the bag (2), ensuring optimal product presentation and minimizing material distortion due to gravitational settling.

The absorbent article herein is typically a folded absorbent article, preferably a folded diaper and/or pant, and wherein a crotch portion (11) of the absorbent article is oriented towards the top of the bag, and wherein a rear and front end (12) of the absorbent article are oriented towards the bottom of the bag. This configuration aids in maintaining the folded integrity of the absorbent articles and reduces creasing or compression-related defects. Additionally, this arrangement can improve the ease of withdrawal for the consumer.

Preferably, the film is substantially airtight and/or non-breathable, and preferably substantially translucent. This provides a dual advantage: first, by preventing external moisture ingress, it helps preserve the absorbency and structural integrity of the contained absorbent articles; second, the translucent nature of the film enables visual inspection of the packaged products, aiding in quality control and consumer convenience.

The composite material is preferably substantially free of adhesive or adhesive polymer for providing adhesion between the film and the nonwoven fabric and is preferably free of laminates of film and nonwoven. The absence of adhesives and laminates reduces the risk of delamination over time, enhances recyclability, and maintains the mechanical properties of the composite material. Alternative adhesion methods, such as thermal bonding or mechanical interlocking, may be employed to achieve the necessary structural cohesion without compromising sustainability objectives.

### THE METHOD OF MANUFACTURE

A further aspect of the disclosure relates to method for manufacture of a package for a stack of absorbent articles, the method comprising the, preferably sequential, steps of:
i. co-forming, preferably co-extruding, a film and nonwoven to form a composite material;
ii. shaping the composite material into a bag having an open end (12) and a first closed end (6);
iii. filling the bag with a stack of absorbent articles (3) through the open end (12); and
iv. sealing the open end to form a second closed end (7), such to contain the stack of absorbent articles between first and second closed ends. Advantageously such a method allows for a package with improved softness to the touch perception by a user whilst allowing to reduce scope 3 emissions.

The co-forming step can be selected from any one of the following: cast extrusion, blown film extrusion, extrusion-coating, extrusion-lamination, curtain coating extrusion, profile extrusion, filament spinning and/or spun-melt nonwoven extrusion; preferably cast extrusion, blown film extrusion, profile extrusion, filament spinning and/or spun-melt nonwoven extrusion.

The package may comprise any of the features of the preferred embodiments described above, for example with regards to material composition of the film/nonwoven, basis weights etc.

### TEST METHODS

### Test Method: Opacity

Opacity by contrast ratio measurements are made using a 0°/45° spectrophotometer suitable for making standard Hunter L*a*b* color measurements (e.g. Hunterlab Labscan XE spectrophotometer, Hunter Associates Laboratory Inc., Reston Va. or equivalent). The diameter of the instrument's measurement port should be chosen such that only the region of interest is included within the measurement port. Analyses are performed in a room controlled at about 23° C.±2 C.° and 50%±2% relative humidity. Samples are conditioned at the same condition for 2 hours before testing.

Calibrate the instrument per the vender instructions using the standard black and white tiles provided by the vendor. Set the spectrophotometer to use the CIE XYZ color space, with a D65 standard illumination and 10° observer. Using cryogenic spray and scissors excise the topsheet specimen from the article for testing. Place the specimen flat against the instrument with the body facing surface toward the spectrophotometer's measurement port and the region of interest within the port. Place the white standard tile onto the opposing surface of the specimen such that it completely covers the measurement port. Take a reading for XYZ and record to 0.01 units. Without moving the specimen, remove the white plate and replace it with the black standard plate. Take a second reading for XYZ and record to 0.01 units. Repeat this procedure at a corresponding site for a total of ten (10) replicates specimens.

Opacity is calculated by dividing the Y value measured using the black tile as backing, divided by the Y value measured using the white tile as backing, then multiplying the ratio by 100. Record opacity to the nearest 0.01%. Calculate opacity for the 10 replicates and report the average opacity to the nearest 0.01%.

### EXAMPLES

### Example 1

### Base Polymer: Polypropylene

The composition comprises the following layers:
1. **Extensional Thinning Layer:**
   This layer consists of a polypropylene (PP) homo-polymer or co-polymer with a melt flow index (MFI) ranging from 2 to 30. The layer has a thickness of 0.25 to 6 µm. This configuration ensures efficient extensional thinning, contributing to the overall flexibility and processability of the composite material.
2. **Extensional Thickening Layer:**
   This layer is composed of long-branched high melt strength polypropylene or in-situ long-branched polypropylene obtained through crosslinking, with an MFI ranging from 2 to 30. The extensional thickening layer is blended with a hydrocarbon tackifier that is fully compatible with the base polymer. The hydrocarbon tackifier is preferably selected from compounds having an average molecular weight of 1,000 to 3,000. The tackifier is present in a proportion of 15% to 60% by weight, more preferably from 20% to 40% by weight of the total weight of the extensional thickening layer. The layer thickness ranges from 0.1 to 6 µm. This composition enhances mechanical integrity, providing improved tear resistance and puncture strength while maintaining processability.

This composition is particularly suitable for coated substrates used in the production of non-breathable bags for packaging applications described herein. Its optimized layer structure ensures a balance of strength, flexibility, and barrier properties, making it ideal for protecting moisture-sensitive products.
Product: PP spun-bond non-woven
Equipment: Reicofil bi-component spun-bond machine.
Layer structure: core/sheath filament A/B
Core layer A: 90% by weight High Melt Strength Polypropylene Daploy WS420 HMS MFI 22+ 10% by weight Eastman tackifier Plastolyn R1140.
Sheath layer B: Polypropylene Homo-polymer Repsol Isplen PP086Y3E MFI 25.
Process settings at stable running:
   Extrusion temperature: 260°C
   Spinneret capillary diameter sheath: 0.6mm
   Spinneret capillary diameter core: 0.3 mm
   Line speed 300m/min
   Resulting filament denier: 0.35
   Draw down ratio: 105

## Claims

1. A package (1) comprising a bag (2) and a stack of absorbent articles (3) wherein the stack of absorbent articles is arranged in the bag, wherein the bag (2) is made of a composite material, said composite material comprising, preferably consisting essentially of, even more preferably consisting of, a film and at least a layer of nonwoven fabric, wherein the film forms an inner layer (4) of the bag (2) for contact with the absorbent articles (3), and the nonwoven fabric forms an outer layer (5) of the bag (3), and wherein the composite material is substantially free of an adhesive layer between said film and said nonwoven, preferably wherein the film and nonwoven are substantially co-formed.

2. A package according to Claim 1, wherein the film and nonwoven are co-formed, preferably extrusion co-formed, such that the nonwoven directly adjoins the film.

3. A package according to any of the preceding Claims, wherein the nonwoven fabric comprises, preferably consists of, one or more layers; and wherein the nonwoven fabric has a basis weight from 5 to 50 gsm, preferably from 5 to 40 gsm, more preferably from 6 to 30 gsm, even more preferably from 7 to 20 gsm, most preferably from 8 to 10 gsm.

4. A package according to any of the preceding Claims, wherein the film has a basis weight of less than 10 gsm, preferably less than 8 gsm, more preferably from 1 gsm to 5 gsm, even more preferably from 2 gsm to 4 gsm.

5. A package according to any of the preceding Claims, wherein the nonwoven comprises fibers selected from the group consisting of polyolefin fibers, polyester fibers, and mixtures thereof; preferably wherein the nonwoven comprises fibers selected from polyethylene fibers, polypropylene fibers, polylactic acid fibers, and/or Polyethylene terephthalate fibers.

6. A package according to any of the preceding Claims, wherein the film comprises a copolymer of ethylene and methyl acrylate.

7. A package according to any of the preceding Claims, wherein the film is essentially free of polyethylene.

8. A package according to any of the preceding Claims, wherein the composite material has a total basis weight of from 7 gsm to 30 gsm, preferably from 8 gsm to 25 gsm, even more preferably from 9 gsm to 20 gsm, even more preferably from 10 gsm to 15 gsm, even more preferably from 11 gsm to 14gsm.

9. A package according to any of the preceding Claims, wherein the bag (2) has a first closed end (6), a second closed end (7), and a peripheral wall (8) between said bottom and said top.

10. A package according to Claim 9, wherein the peripheral wall (8) comprises a front wall, a rear wall opposite said front wall, a first side wall, and a second side wall opposite said first side wall, and wherein at a top position, the first and the second side wall are folded inwardly such that two first wing portions (9) are formed where the top joins the first side wall and such that two second wing portions (10) are formed where the top joins the second side wall.

11. A package according to any of Claims 9 to 10, wherein the stack of absorbent articles is arranged such that each absorbent article (3) extends in an upright direction between the first closed end (6) and the second closed end (7).

12. A package of any of the Claims 8 to 11, wherein the absorbent article is a folded absorbent article, preferably a folded diaper and/or pant, and wherein a crotch portion (11) of the absorbent article is oriented towards the top of the bag, and wherein a rear and front end (12) of the absorbent article are oriented towards the bottom of the bag.

13. A package according to any of the preceding Claims, wherein the film is substantially airtight and/or non-breathable, and preferably substantially translucent.

14. A Package according to any of the preceding Claims, wherein the composite material is substantially free of adhesive or adhesive polymer for providing adhesion between the at film and the nonwoven fabric, and is preferably free of laminates of film and nonwoven.

15. A method for manufacture of a package for a stack of absorbent articles, the method comprising the, preferably sequential, steps of:
i. co-forming, preferably co-extruding, a film and nonwoven to form a composite material;
ii. shaping the composite material into a bag having an open end (12) and a first closed end (6);
iii. filling the bag with a stack of absorbent articles (3) through the open end (12); and
iv. sealing the open end to form a second closed end (7), such to contain the stack of absorbent articles between first and second closed ends.

16. A method according to Claim 15 wherein the package comprises one or more of the features according to the package of any of Claims 1 to 14.
